# EUROPÄISCHE PATENTSCHRIFT

(11) **EP 1 782 556 B1**
(45) Veröffentlichungstag und Bekanntmachung des Hinweises auf die Patenterteilung: **20.02.2013**
(21) Anmeldenummer: 05778307.8
(22) Anmeldetag: 14.07.2005
(51) Int. Cl.: H04B 7/26

(54) **VORRICHTUNG ZUR MOBILEN DATENÜBERTRAGUNG**
DEVICE FOR MOBILE TRANSMISSION OF DATA
DISPOSITIF DE TRANSMISSION DE DONNÉES MOBILE

(30) Priorität: 26.07.2004 DE 202004011696 U
(43) Veröffentlichungstag der Anmeldung: 09.05.2007
(73) Patentinhaber: Deutsche Telekom AG, 53113 Bonn (DE)
(72) Erfinder: NIEDERAU, Dirk, 53639 Königswinter-Ittenbach (DE)
(74) Vertreter: Riebling, Peter
(86) Internationale Anmeldenummer: PCT/DE2005/001254
(87) Internationale Veröffentlichungsnummer: WO 2006/012835

(56) Entgegenhaltungen:
- US-A1- 2003 001 743
- US-A1- 2004 046 637

## Beschreibung

Die Erfindung betrifft eine Vorrichtung zur mobilen Datenübertragung nach dem Oberbegriff des Anspruchs 1.

Moderne Telemetriesysteme zur Übertragung von mobil entstehenden Daten, wie sie z.B. im Radsport oder Autorennsport erfasst werden, nutzen eine Datenübertragung per Funk zwischen einer datengenerierenden Stelle (Fahrzeug) und einer datenverarbeitenden (zentralen) Stelle.

Dabei werden eigens entwickelte Funksysteme eingesetzt, die auf einer speziellen Frequenz arbeiten und eine begrenzte Reichweite aufweisen. Es kommt oft vor, dass bei schlechten Ausbreitungsbedingungen für die Funksignale oder zu großem Abstand zwischen Sender und Empfänger keine kontinuierliche Datenübertragung möglich ist.

Im Radsport kommen z.B. Funkanlagen zum Einsatz, die bei einer Frequenz von 433 MHz arbeiten. Der Radsportler besitzt eine Sendeeinheit, die verschiedene fahrdynamische Daten und körperliche Daten des Fahrers erfasst und per Funk an eine Empfangseinheit sendet. Die Empfangseinheit befindet sich in einem Begleitfahrzeug, wo die Daten überwacht und ausgewertet werden. Je nach Streckenverhältnissen und Ausbreitungsbedingungen für die Funksignale ist im Begleitfahrzeug nicht immer ein kontinuierlicher Empfang der vom Radfahrer ausgesendeten Daten gewährleistet.

Die US 2004/0046637 A1 oder die US 2003/0001743 A1 offenbarten eine Vorrichtung zur mobilen Datenübertragung über ein öffentliches digitales Mobilkommunikationssystem, welche mindestens eine mobile Einrichtung mit Einrichtungen zur Erfassung von physikalischen Größen und Umwandlung der physikalischen Größen in digitale Daten umfasst und eine zu dem öffentlichen digitalen Mobilkommunikationssystem kompatiblen Sendeeinrichtung oder Sende-Empfangseinrichtung zur Übertragung der digitalen Daten über das Mobilkommunikationssystem an mindestens eine zentrale Einrichtung. Die zentrale Einrichtung besitzt eine zu dem öffentlichen digitalen Mobilkommunikationssystem kompatiblen Empfangseinrichtung oder Sende-Empfangseinrichtung zum Empfang der digitalen Daten, eine Datenverarbeitungseinrichtung zur Speicherung, Verarbeitung und Auswertung der empfangene digitalen Daten, und eine Mensch-Maschine-Schnittstelle zur Eingabe von Steuerbefehlen und Ausgabe der verarbeiteten digitalen Daten.

Es ist die Aufgabe der Erfindung, eine Vorrichtung zur mobilen Datenübertragung anzugeben, die eine sichere mobile Datenübertragung zwischen einem oder mehreren mobilen Sendeeinheiten und mindestens einer Empfangseinheit gewährleistet. Ferner soll durch die Empfangseinheit eine individuelle Steuerung und Verwaltung der Sendeeinheiten möglich sein.

Diese Aufgabe wird erfindungsgemäß durch die Merkmale des Anspruchs 1 gelöst.

Bevorzugte Ausgestaltungen und andere vorteilhafte Merkmale der Erfindung sind in den abhängigen Patenansprüchen angegeben.

Die erfindungsgemäße Vorrichtung ist geeignet, von einer oder mehreren mobilen Einrichtungen erfasste Daten (Messgrößen) über eine entsprechende Sendeeinrichtung oder Sende-Empfangseinrichtung über ein öffentliches digitales Mobilkommunikationsnetz an eine entsprechende zentrale Empfangseinrichtung oder Sende-Empfangseinrichtung zu übertragen.

Ferner, können eine oder mehrere mobile Einrichtungen von der zentralen Einrichtung (fern)gesteuert werden, indem entsprechende Steuersignale an die mobile Einrichtung übertragen werden. Durch die variable, vorzugsweise automatisierte Ansteuerung der mobilen Einrichtung ist es möglich, von der mobilen Einrichtung erfasste telemetrische Daten über einen längeren Zeitraum zu übertragen.
Der Einssatz eines Mobilkommunikationssystems zur Datenübertragung ermöglicht eine sehr zuverlässige, flächendeckende Datenübertragung zur zentralen Einrichtung und/oder beliebigen anderen kompatiblen Empfangseinrichtungen.

Das beschriebene System lässt sich insbesondere im Sport zur Erfassung und Übertragung von technik- und/oder personenbezogenen Messdaten einsetzen, z.B. die Übertragung von Herzfrequenzwerten, die anschließend im TV oder Internet zur Anzeige gebracht werden können.

Um mögliche Anwendungen, insbesondere Anwendungen im Sport gerecht zu werden, ist das Verhältnis von Gewicht der mobilen Einrichtung und maximal möglichen Datenübertragungszeitraum optimiert, beispielsweise:
- Gewicht weniger als 100g
- Datenübertragung bis zu 8h
- Mobile Einrichtung ist wasserdicht

Ein weiterer Vorteil der Erfindung ist, dass von der mobilen Einrichtung die Daten an jedes mobilfunkkompatible Empfangsgerät übermittelbar sind, einfach durch entsprechende Anwahl der Mobilfunkrufnummer bzw. IP-Adresse der empfangenden (zentralen) Einrichtung.

Innerhalb Europas ist eine nahezu flächendeckende, einheitliche Mobilfunkversorgung durch GSM- bzw. UMTS-Mobilfunknetze gewährleistet. Da ein solches Mobilkommunikationssystem zur Übermittelung der Daten eingesetzt wird, spielt die Entfernung zwischen mobiler Einheit und zentraler Einheit keine Rolle.

Ein Ausführungsbeispiel der Erfindung ist nachfolgend anhand der Zeichnung beschrieben.

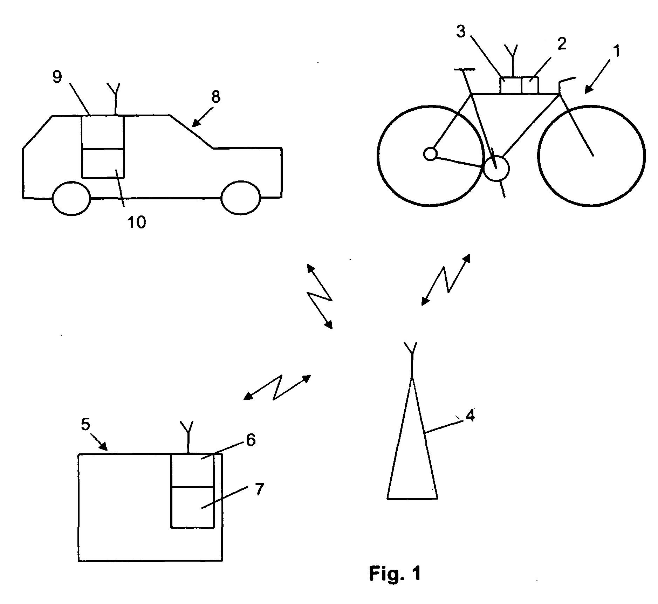

In Figur 1 ist eine mögliche Anwendung im Profi-Radsport für die dort mobil entstehenden Leistungsdaten eines Radsportlers dargestellt. Die Fahrdaten und körperlichen Daten eines Radsportlers werden mit einem am Fahrrad 1 montierten Radcomputer 2 erfasst und können z.B. über eine serielle Schnittstelle an ein Mobilkommunikationsendgerät 3 übertragen werden. Mittels des Radcomputers 2 und damit verbundenen diversen Messinstrumenten, wie z.B. Tachometer, Kilometerzähler, Trittfrequenzmesser, Herzfrequenzmesser, werden entsprechende physikalische Messwerte, wie z.B. Geschwindigkeit, gefahrene Kilometer, Herzfrequenz, Leistung (Wattzahl) erfasst und in digitale Daten umgewandelt. Die digitalen Daten können direkt am Radcomputer 2 angezeigt werden und über die serielle Schnittstelle drahtlos (IR, Bluetooth) oder drahtgebunden an andere Geräte, z.B. das Mobilkommunikationsendgerät 3, übertragen werden. Von dort werden die erfassten Daten über ein öffentliches Mobilkommunikationsnetz, vereinfacht dargestellt durch eine Basisstation 4, an einen Empfänger 6 in einer zentrale Einrichtung 5 übertragen. Vom Empfänger 6 werden die empfangenen Daten an eine Datenverarbeitungseinrichtung 7 weitergeleitetet und können dort verarbeitet und ausgegeben werden. Ebenfalls ist eine entsprechende Übertragung der Daten an einen in einem Begleitfahrzeug 8 installierten Empfänger 9 möglich, wo die Daten dann in einer Auswerteeinrichtung 10 weiterverarbeitet werden können.

Die Übertragung der Daten von der mobilen Einrichtung 1, 2, 3 zur zentralen Einrichtung 5 erfolgt vorzugsweise über den Paketnachrichtendienst GPRS, da mit diesem Verfahren eine permanente Verbindung zwischen Sendeeinrichtung und Empfangseinrichtung möglich ist, ohne die Funkschnittstelle während Pausen und Unterbrechungen in der Datenübertragung unnötig zu belasten.

Ausgehend von der zentralen Einrichtung 5 und/oder dem Begleitfahrzeug 8 kann die mobile Einrichtung, das heißt das Mobilkommunikationsendgerät 3 und der Radcomputer 2, in ihrem Verhalten fern-gesteuert werden. Insbesondere ist bei einem Einsatz von mehreren mobilen Einrichtungen eine Optimierung des jeweiligen Datenübertragungszeitraumes zur variablen Ansteuerung der einzelnen mobilen Sendeeinrichtungen 3 und dem Radcomputer 2 möglich.
Ferner kann die zentrale Einrichtung 5 oder das Begleitfahrzeug 8 die Einsatzdauer der jeweiligen mobilen Sendeeinrichtung 3, die übertragene Datenmenge und Sendedauer -/-häufigkeit durch vorgegebene Schwellwerte oder Steuersignale verändern und steuern, um z.B. die Leistungsaufnahme der mobilen Einrichtungen zu minimieren und eine maximale Sendedauer zu gewährleisten.

Durch die zentrale Einrichtung 5 und/oder das Begleitfahrzeug 8 kann ferner eine Steuerung für selektive/variable Datenbereitstellung der Daten mehrerer mobiler Einrichtungen erfolgen. Das heißt die Datenbereitstellung und -übertragung kann nach Sendern und/oder Datenart selektiert bzw. beschränkt werden.

Die Daten können von der zentralen Einrichtung 5 unmittelbar an andere Instanzen weitergeleitet werden und dort im TV für Live-Übertragungen oder im Internet bereit gestellt werden.

## Patentansprüche

1. Vorrichtung zur mobilen Datenübertragung über ein öffentliches digitales Mobilkommunikationssystem (4), welche umfasst:
mindestens eine mobile Einrichtung mit
Einrichtungen (2) zur Erfassung von physikalischen Größen und Umwandlung der physikalischen Größen in digitale Daten; und
einer zu dem öffentlichen digitalen Mobilkommunikationssystem kompatiblen Sendeeinrichtung oder Sende-Empfangseinrichtung (3) zur Übertragung der digitalen Daten über das Mobilkommunikationssystem an mindestens eine zentrale Einrichtung (5);
eine zentrale Einrichtung (5; 8) mit
einer zu dem öffentlichen digitalen Mobilkommunikationssystem kompatiblen Empfangseinrichtung oder Sende-Empfangseinrichtung (6; 9) zum Empfang der digitalen Daten,
einer Datenverarbeitungseinrichtung (7; 10) zur Speicherung, Verarbeitung und Auswertung der empfangene digitalen Daten, und einer Mensch-Maschine-Schnittstelle zur Eingabe von Steuerbefehlen und Ausgabe der verarbeiteten digitalen Daten,
wobei die zentrale Einrichtung (5; 8) Mittel zur Übertragung der Steuerbefehle auf die mobile Einrichtung (2, 3) und die mobile Einrichtung Mittel (3) zum Empfang und zur Auswertung der Steuerbefehle aufweist,
wobei die Steuerbefehle Befehle zur Steuerung der Einsatzdauer der mobilen Einrichtung (2, 3), zur Sendedauer und -häufigkeit, zur Steuerung der Art und Menge der zu übertragenden Daten enthalten, und Befehle zur Selektion mindestens einer ausgewählten mobilen Einrichtung (2, 3).

2. Vorrichtung nach Anspruch 1, **dadurch gekennzeichnet, dass** die mobile Einrichtung ein Mobiltelefon (3) umfasst.

3. Vorrichtung nach einem der vorhergehenden Ansprüche, **dadurch gekennzeichnet, dass** die digitalen Daten per Kurznachricht, SMS, über das Mobilkommunikationssystem (4) übertragen werden.

4. Vorrichtung nach einem der vorhergehenden Ansprüche, **dadurch gekennzeichnet, dass** die digitalen Daten per Paketnachrichtendienst, GPRS: General Packet Radio Service, über das Mobilkommunikationssystem (4) übertragen werden.

5. Vorrichtung nach einem der vorhergehenden Ansprüche, **dadurch gekennzeichnet, dass** das Mobilkommunikationssystem (4) ein GSM- und/oder UMTS-Mobilkommunikationssystem ist.

6. Vorrichtung nach einem der vorhergehenden Ansprüche, **dadurch gekennzeichnet, dass** die mobile Einrichtung (2, 3) bei vorgegebenem Gewicht eine bestimmte minimalen Sendedauer und/oder eine bestimmte minimale übertragene Datenmenge einhält.

## Claims

1. Apparatus for mobile data transmission via a public digital mobile communications system (4), which comprises:
at least one mobile device having
devices (2) for the detection of physical quantities and conversion of the physical quantities to digital data; and
a transmitting device or transmitting/receiving device (3) compatible with the public digital mobile communications system for transmission of the digital data via the mobile communications system to at least one central facility (5);
a central facility (5; 8) having
a receiving device or transmitting/receiving device (6; 9) compatible with the public digital mobile communications system for reception of the digital data, a data processing device (7; 10) for the storage, processing and evaluation of the digital data received, and
a human/machine interface for the input of control commands and output of the processed digital data,
wherein the central facility (5; 8) has means for transmission of the control commands to the mobile device (2, 3) and the mobile device has means (3) for the reception and
evaluation of the control commands, wherein the control commands contain commands for control of the duration of use of the mobile device (2, 3), the duration and frequency of transmission, for control of the type and quantity of data to be transmitted, and commands for the selection of at least one selected mobile device (2, 3).

2. Apparatus according to claim 1, **characterised in that** the mobile device comprises a mobile telephone (3).

3. Apparatus according to one of the preceding claims, **characterised in that** the digital data are transmitted by short message, SMS, via the mobile communications system (4).

4. Apparatus according to one of the preceding claims, **characterised in that** the digital data are transmitted by packet message service, GPRS (general packet radio service), via the mobile communications system (4).

5. Apparatus according to one of the preceding claims, **characterised in that** the mobile communications system (4) is a GSM and/or UMTS mobile communications system.

6. Apparatus according to one of the preceding claims, **characterised in that** the mobile device (2, 3) with a predetermined weight complies with a given minimum transmission time and/or a given minimum quantity of data transmitted.

## Revendications

1. Dispositif pour la transmission de données mobile par l'intermédiaire d'un système de communication mobile numérique public (4), qui comprend :
au moins un appareil mobile avec
des appareils (2) pour détecter des grandeurs physiques et convertir ces grandeurs physiques en données numériques ; et
un émetteur ou émetteur-récepteur (3), compatible avec le système de communication mobile numérique public, pour transmettre des données numériques à au moins un appareil central (5) par l'intermédiaire du système de communication mobile ;
un appareil central (5 ; 8) avec
un récepteur ou émetteur-récepteur (6 ; 9), compatible avec le système de communication mobile numérique public, pour recevoir les données numériques,
un appareil de traitement de données (7 ; 10) pour stocker, traiter et analyser les données numériques reçues, et
une interface homme-machine pour entrer des ordres de commande et sortir les données numériques traitées,
étant précisé que l'appareil central (5 ; 8) comporte des moyens pour transmettre les ordres de commande à l'appareil mobile (2, 3) et que l'appareil mobile comporte des moyens (3) pour recevoir et analyser les ordres de commande,
étant précisé que les ordres de commande contiennent des ordres pour la commande de la durée d'utilisation de l'appareil mobile (2, 3), pour la durée et la fréquence d'émission, pour la commande du type et de la quantité des données à transmettre, et des ordres pour la sélection d'au moins un appareil mobile (2, 3) choisi.

2. Dispositif selon la revendication 1, **caractérisé en ce que** l'appareil mobile comprend un téléphone mobile (3).

3. Dispositif selon l'une des revendications précédentes, **caractérisé en ce que** les données numériques sont transmises par message court, SMS, par l'intermédiaire du système de communication mobile (4).

4. Dispositif selon l'une des revendications précédentes, **caractérisé en ce que** les données numériques sont transmises par un service de communication par paquets, GPRS : General Packet Radio Service, par l'intermédiaire du système de communication mobile (4).

5. Dispositif selon l'une des revendications précédentes, **caractérisé en ce que** le système de communication mobile (4) est constitué par un système de communication mobile GSM et/ou UMTS.

6. Dispositif selon l'une des revendications précédentes, **caractérisé en ce que** l'appareil mobile (2, 3) contient pour un poids prédéfini une durée d'émission minimale définie et/ou une quantité de données transmise minimale définie.
